(19) Europäisches Patentamt
European Patent Office
Office européen des brevets

(11) EP 3 777 920 A1

(12) EUROPEAN PATENT APPLICATION

(43) Date of publication:
17.02.2021 Bulletin 2021/07

(21) Application number: 20197505.9

(22) Date of filing: 11.11.2016

(51) Int Cl.:
A61M 1/36 (2006.01)     B01D 15/08 (2006.01)
B01J 20/02 (2006.01)     B01J 20/06 (2006.01)
B01J 20/28 (2006.01)     B01J 20/32 (2006.01)
C01F 17/00 (2020.01)     C01G 23/047 (2006.01)
B01D 15/12 (2006.01)     C01F 17/247 (2020.01)

(84) Designated Contracting States:
AL AT BE BG CH CY CZ DE DK EE ES FI FR GB
GR HR HU IE IS IT LI LT LU LV MC MK MT NL NO
PL PT RO RS SE SI SK SM TR

(30) Priority: 30.11.2015  JP 2015232770
30.11.2015  JP 2015232771

(62) Document number(s) of the earlier application(s) in
accordance with Art. 76 EPC:
16870417.9 / 3 384 941

(71) Applicant: Toray Industries, Inc.
Tokyo, 103-8666 (JP)

(72) Inventors:
• HAN, Aishan
Otsu-shi, Shiga 520-8558 (JP)

• IWAKAWA, Sari
Otsu-shi, Shiga 520-8558 (JP)
• UENO, Yoshiyuki
Otsu-shi, Shiga 520-8558 (JP)

(74) Representative: Mewburn Ellis LLP
Aurora Building
Counterslip
Bristol BS1 6BX (GB)

Remarks:
This application was filed on 22-09-2020 as a
divisional application to the application mentioned
under INID code 62.

(54) PHOSPHORUS ADSORBENT, POROUS FIBER AND PHOSPHORUS ADSORPTION COLUMN

(57) The purpose of the present invention is to provide: a phosphorus adsorbent which is to be used for adsorbing phosphorous in extracorporeal circulation with little fear of disturbing the environment *in vivo;* a phosphorus adsorption column which comprises the phosphorus adsorbent disposed therein; a porous fiber which comprises the phosphorus adsorbent carried therein with little fear of disturbing the environment *in vivo;* and a phosphorus adsorption column which comprises the porous fiber disposed therein. To achieve the purpose as described above, the present invention has the following constitution. Namely, the phosphorus adsorbent, which is to be used for adsorbing phosphorus in extracorporeal circulation, is a powder comprising a carbonate of neodymium and having a solubility in 100 g of water at 20 °C of 10 mg or less. The porous fiber, which comprises the phosphorus adsorbent carried therein, shows a change in pH value of from -1.0 to +1.0 inclusive before and after stirring in physiological saline for 4 hours. The phosphorus adsorption columns respectively comprise the phosphorus adsorbent and the porous fiber each disposed therein.

**Description**

TECHNICAL FIELD

The present invention relates to a phosphorus adsorbent, a porous fiber, and a phosphorus adsorption column.

BACKGROUND ART

Patients with impaired renal function are often affected with hyperphosphatemia due to reduced phosphorus excretion. Hyperphosphatemia produces severe abnormalities in the metabolism of calcium and phosphorus, and may cause renal osteodystrophy due to decrease in the serum calcium level, acceleration of production and secretion of parathyroid hormone (PTH), ectopic calcification, and suppression of vitamin D activation. Even if a patient switches to the dialysis therapy due to renal failure, the above-mentioned clinical conditions will persist unless phosphorus homeostasis is maintained. Therefore, treatment of hyperphosphatemia is indispensable for both dialysis patients and pre-dialysis patients with renal failure. Currently known therapies of hyperphosphatemia include diet therapy and a method based on an oral phosphorus adsorbent. In the treatment of hyperphosphatemia in dialysis patients and pre-dialysis patients with renal failure, diet therapy is inadequate, and a method based on an oral phosphorus adsorbent is required.

As an oral phosphorus adsorbent, inorganic and organic oral phosphorus adsorbents are known.

As an inorganic oral phosphorus adsorbent, aluminum salts and calcium salts have been widely used. The ingested aluminum and calcium combine with the intestinal phosphoric acid to form insoluble phosphates, so that the absorption of phosphorus is inhibited. Administration of aluminum salts, however, causes accumulation of aluminum in the body, which may cause brain diseases, osteomalacia and the like. Calcium absorption may also cause hypercalcemia, and may quicken the death of patients due to calcium deposition to the aortas or ectopic calcification.

In this context, as an inorganic oral phosphorus adsorbent that does not raise the calcium level, lanthanum carbonate is known. However, intestinal absorption of lanthanum and accumulation of lanthanum in bone have been recognized, and concern has been expressed in terms of long-term safety (Non-Patent Document 1).

Meanwhile, Patent Document 1 discloses a lanthanoid carbonate as an inorganic oral phosphorus adsorbent. However, like the lanthanum carbonate, the lanthanoid carbonate can easily release metal ions in an atmosphere of a strong acid such as gastric acid, although it has low water solubility under neutral conditions. Therefore, the lanthanoid carbonate has a problem of accumulation of lanthanoids in bone due to long-term use.

Meanwhile, as an organic oral phosphorus adsorbent, sevelamer hydrochloride, which is a type of polyallylamine, is known. Sevelamer hydrochloride is widely used in the treatment of hyperphosphatemia because it does not cause the above-mentioned side effects associated with inorganic salts. The dosage of sevelamer hydrochloride is usually set to 3 to 6 g/day, or at most 9 g/day, since it is necessary to administer a high dose of sevelamer hydrochloride in order to remarkably reduce the absorption of phosphoric acid. Since sevelamer hydrochloride swells by the absorption of water in the gastrointestinal tract, it causes problems such as constipation, abdominal pain, and abdominal distension, and there are also reports of serious side effects such as intestinal perforation and intestinal obstruction (Non-Patent Document 2) . Moreover, since sevelamer hydrochloride inhibits the absorption of fat-soluble vitamins (vitamin D, vitamin E, and vitamin K) and folates, these nutrients have to be supplemented in long-term administration of sevelamer hydrochloride.

Moreover, considering that the oral phosphorus adsorbent must be taken continuously in combination with lifetime dialysis treatment, it is not desirable in view of the quality of life (QOL) of patients that the oral phosphorus adsorbent may have side effects such as increase in the blood calcium level, constipation, and abdominal distension as described above depending on the type of the oral preparation used.

Therefore, it is meaningful to develop a novel form of hyperphosphatemia therapy that is completely different from oral medicine.

Patent Document 2 describes that a phosphorus adsorbent is supported on a porous carrier to form a molded article intended for extracorporeal circulation, and that the shape of the molded article may be any shape suitable for the usage, such as a granular shape, a thread shape, and a hollow fiber shape. Patent Document 2 discloses a hydrated oxide of a rare earth element as the phosphorus adsorbent. Specifically, the document discloses an example in which cerium hydroxide is added to polyethylene-vinyl alcohol, and the resultant mixture is formed into granules and packed in a column for the evaluation of the phosphorus adsorption capability.

Patent Document 3 describes combination use of a phosphorus adsorption column and a dialyzer. Patent Document 3 discloses a polycationic polymer having a specific structure as a phosphorus adsorbent.

PRIOR ART DOCUMENTS

NON-PATENT DOCUMENTS

Non-Patent Document 1: Takashi Shigematsu et al., "Bone biopsy test", data on file of Bayer Yakuhin, Ltd., 2009
Non-Patent Document 2: Kazuhiko Ishikiriyama et al., JOURNAL OF COLLOID AND INTERFACE SCIENCE, 1995, vol. 171, pp. 103-111

PATENT DOCUMENTS

Patent Document 1: Japanese Patent Laid-open Publication (Translation of PCT Application) No. 11-503119
Patent Document 2: Japanese Patent Laid-open Publication No. 61-004529
Patent Document 3: Japanese Patent Laid-open Publication No. 2002-102335

SUMMARY OF THE INVENTION

PROBLEMS TO BE SOLVED BY THE INVENTION

However, the hydrated oxide of a rare earth element, which is a phosphorus adsorbent supported on a porous carrier in Patent Document 2, is not preferable for use in which the phosphorus adsorbent is supported in a porous fiber, because the phosphorus adsorbent is large in the pH change and greatly disturbs the biological environment.
Similarly, in the phosphorus adsorption column described in Patent Document 3, the polycationic polymer having a specific structure, which is used as a phosphorus adsorbent, is large in the pH change and may possibly disturb the pH balance of blood, that is, the biological environment, because it adsorbs phosphorus in exchange for hydrochloric acid.
In view of the above-mentioned situation, an object of the present invention is to provide a phosphorus adsorbent for use in phosphorus adsorption in extracorporeal circulation, which may scarcely disturb the biological environment, and a phosphorus adsorption column containing the phosphorus adsorbent incorporated therein, and to provide a porous fiber in which a phosphorus adsorbent is supported, which may scarcely disturb the biological environment, and a phosphorus adsorption column containing the porous fiber incorporated therein.

SOLUTIONS TO THE PROBLEMS

In the present invention, as a result of intensive studies to solve the above-mentioned problems, the following invention has been completed.

(1) A phosphorus adsorbent for use in phosphorus adsorption in extracorporeal circulation, containing a carbonate of a rare earth element or a Group 4 oxide, and being a granule having a solubility of 10 mg or less in 100 g of water at 20°C.
(2) The phosphorus adsorbent according to (1), wherein the rare earth element is selected from the group consisting of lanthanum, cerium, praseodymium, and neodymium.
(3) The phosphorus adsorbent according to (1) or (2), being a granule having an average particle diameter more than 100 nm and 100 $\mu$m or less.
(4) The phosphorus adsorbent according to any one of (1) to (3), having a phosphorus adsorption capability of 4.0 mg/g or more.
(5) A porous fiber in which a phosphorus adsorbent is supported, having a pH change of -1.0 or more and +1.0 or less after being stirred for 4 hours in physiological saline.
(6) The porous fiber according to (5), having a phosphorus adsorption capability of 3.0 mg/g or more.
(7) The porous fiber according to (5) or (6), wherein the phosphorus adsorbent is a granule, and the granule has a solubility of 10 mg or less in 100 g of water at 20°C in a state of being supported in the porous fiber.
(8) The porous fiber according to any one of (5) to (7), wherein the phosphorus adsorbent is a granule, and the granule has an average particle diameter more than 100 nm and 100 $\mu$m or less.
(9) The porous fiber according to any one of (5) to (8), having a diameter of 50 to 1000 $\mu$m.
(10) The porous fiber according to any one of (5) to (9), having an average pore radius of 0.5 to 100 nm, and a specific surface area of pores of 10 m$^2$/g or more.

(11) The porous fiber according to any one of (5) to (10), wherein the phosphorus adsorbent contains a salt of a rare earth element or a Group 4 oxide.

(12) The porous fiber according to (11), wherein the phosphorus adsorbent contains a carbonate of a rare earth element, and the rare earth element is selected from the group consisting of lanthanum, cerium, praseodymium, and neodymium.

(13) A phosphorus adsorption column, containing the phosphorus adsorbent according to any one of (1) to (4) incorporated therein.

(14) A phosphorus adsorption column, containing the porous fiber according to any one of (5) to (12) incorporated therein.

EFFECTS OF THE INVENTION

The phosphorus adsorbent of the present invention is a phosphorus adsorbent for use in phosphorus adsorption in extracorporeal circulation, which may scarcely disturb the biological environment.

Further, the porous fiber of the present invention is a porous fiber in which a phosphorus adsorbent is supported, which may scarcely disturb the biological environment.

The phosphorus adsorption column of the present invention is a phosphorus adsorption column containing the phosphorus adsorbent of the present invention incorporated therein, or a phosphorus adsorption column containing the porous fiber of the present invention incorporated therein.

EMBODIMENTS OF THE INVENTION

In the following, the phosphorus adsorbent, the porous fiber, and the phosphorus adsorption column of the present invention will be specifically described.

<Phosphorus adsorbent>

In the present invention, the "phosphorus adsorbent" is a compound having a phosphorus adsorption capability, that is, a compound with which a decrease in the phosphorus concentration from a phosphorus starting solution is observed in the measurement of the phosphorus adsorption capability described later.

Among such phosphorus adsorbents, the phosphorus adsorbent of the present invention is a phosphorus adsorbent for use in phosphorus adsorption in extracorporeal circulation, containing a carbonate of a rare earth element or a Group 4 oxide, and being a granule having a solubility of 10 mg or less in 100 g of water at 20°C.

As used herein, the "granule" means particles having an average particle diameter of 1 nm or more and 10 mm or less.

The phosphorus adsorbent of the present invention contains a carbonate of a rare earth element or a Group 4 oxide. The carbonate of a rare earth element is suitable for extracorporeal circulation, because it has high phosphorus adsorptivity but has low solubility in water, and is small in the pH change in physiological saline, so that the phosphorus adsorbent hardly releases inorganic ions when coming into contact with blood.

Among Group 4 oxides, titanium oxide is particularly preferable. Titanium oxide is suitable for extracorporeal circulation, because it has a high phosphorus adsorption capability similarly to the carbonate of a rare earth element, has low solubility in water, and is small in the pH change.

As used herein, the "extracorporeal circulation" means to guide the blood in the living body out of the body, remove predetermined substances (the target in the present invention is a phosphorus compound) from the blood, and return the blood back into the body.

The phosphorus adsorbent of the present invention preferably contains a carbonate of a rare earth element. A phosphorus adsorbent containing a carbonate of a rare earth element tends to be small in the pH change in physiological saline.

In the phosphorus adsorbent of the present invention, the rare earth element is preferably selected from the group consisting of lanthanum, cerium, praseodymium, and neodymium. In this case, the carbonate of a rare earth element is lanthanum carbonate, cerium carbonate, praseodymium carbonate, or neodymium carbonate. These carbonates of rare earth elements are preferable because they have low solubility in water and are small in the pH change in the living body as described later. Neodymium carbonate and cerium carbonate having a high phosphorus adsorption capability are more preferable.

In the phosphorus adsorbent of the present invention, the granule may contain an oxide of a rare earth element, a hydrated oxide of a rare earth element, and the like as components other than the carbonate of a rare earth element or the Group 4 oxide.

The phosphorus adsorbent may be used singly or as a mixture of two or more thereof.

The phosphorus adsorbent of the present invention is a granule having a solubility of 10 mg or less in 100 g of water at 20°C. The solubility is preferably 5 mg or less, more preferably 2 mg or less, particularly preferably 1 mg or less. The solubility of the phosphorus adsorbent of the present invention is measured by the method described later.

The phosphorus adsorbent of the present invention preferably has a phosphorus adsorption capability of 4.0 mg/g or more. The phosphorus adsorption capability is more preferably 4.5 mg/g or more, still more preferably 5.0 mg/g or more, even more preferably 15.0 mg/g or more, especially preferably 70.0 mg/g or more, particularly preferably 100.0 mg/g or more. A phosphorus adsorption capability of the phosphorus adsorbent within the above-mentioned range easily produces a sufficient effect of adsorbing and removing phosphorus in, for example, an extracorporeal circulation column as a final form incorporating therein the phosphorus adsorbent supported in a porous fiber without the need to pack, in the column, a large amount of the porous fiber supporting the phosphorus adsorbent therein. As a result, it is easy to reduce the amount of blood taken out of the body for sufficient phosphorus removal. The phosphorus adsorption capability is preferably 1000.0 mg/g or less, more preferably 800.0 mg/g or less, still more preferably 500.0 mg/g or less. A phosphorus adsorption capability within the above-mentioned range makes it easy to prevent hypophosphatemia that may occur due to an increase in removal of phosphorus, for example. The method of measuring the phosphorus adsorption capability of a phosphorus adsorbent in the present invention is as described later.

The phosphorus adsorbent of the present invention is preferably a granule having an average particle diameter more than 100 nm and 100 $\mu$m or less. The average particle diameter is preferably 100 $\mu$m or less, more preferably 80 $\mu$m or less, particularly preferably 50 $\mu$m or less, since the phosphorus adsorbent is preferably a granule that is as small as possible. An average particle diameter of 100 $\mu$m or less is preferable because, for example, when the phosphorus adsorbent is used in the form of being supported in a porous fiber, the spinneret is hardly clogged during the spinning, and the spinning performance is hardly deteriorated. Moreover, as the average particle diameter is smaller, the specific surface area is larger, and the phosphorus adsorption efficiency is more easily improved. However, if the average particle diameter is smaller than the pore size of the porous fiber, the phosphorus adsorbent may pass through the pores and leak out into a liquid to be treated such as blood. Thus, the average particle diameter is preferably larger than the pore size, and preferably exceeds 100 nm. However, when the granule is an aggregate of primary particles, the size of the primary particles is not limited to the above-mentioned size.

In the present invention, the particle diameter can be measured with a particle counter, a particle size distribution meter or the like by a laser diffraction/scattering method. Using nonspherical particles, the particle diameter and the frequency (%) are plotted on the horizontal axis and the vertical axis, respectively, and values such as volume average particle diameter (MV), number average particle diameter (MN), and area average particle diameter (MA) are obtained as particle diameters. In the present invention, the average particle diameter is determined based on the number average.

One aspect of the phosphorus adsorption column of the present invention is a phosphorus adsorption column containing the phosphorus adsorbent of the present invention incorporated therein. The phosphorus adsorbent used in the phosphorus adsorption column of this aspect may be subjected to secondary processing into, for example, beads, cylinders, or particles having a particle diameter of 100 $\mu$m or more, and then directly packed in the column. As another usage form of the phosphorus adsorbent, beads or the like may be coated with the phosphorus adsorbent. Such a usage form can be preferably used because the phosphorus adsorbent can be easily packed in a large-sized column.

Further, as a form for preventing the phosphorus adsorbent from coming into direct contact with the blood circulating in the extracorporeal blood circuit during dialysis treatment, other than a method of kneading the phosphorus adsorbent into a porous fiber, the phosphorus adsorbent may be dispersed in a dialysis solution, or the phosphorus adsorbent may be packed outside a hollow fiber dialysis membrane.

Further, a method of processing a polymer mixed with the phosphorus adsorbent into a fiber shape is not particularly limited, and examples thereof include a dry spinning method, a wet spinning method, and a dry/wet spinning method combining a dry method and a wet method. The shape of the fiber formed by coagulation depends on the shape of the spinneret used. Major examples of the shape include a hollow fiber shape and a solid fiber shape.

It is preferable that the phosphorus adsorbent of the present invention be used in the state of being supported in the porous fiber of the present invention described later.

<pH>

In general, the pH balance in the living body is very important, and any imbalance of the pH may lead to various diseases. Since the phosphorus adsorbent of the present invention causes a small change in the pH of physiological blood when being brought into contact with blood, the phosphorus adsorbent has little influence on the pH of body fluids such as blood. Herein, a pH change in physiological saline is used as an index for determining whether or not the phosphorus adsorbent is excellent in the pH balance in the living body. Specifically, in a simulation of 4-hour circulation in dialysis, the phosphorus adsorbent is put in physiological saline, stirred at 200 rpm for 4 hours, and a change in the pH before and after the 4-hour stirring is measured. In the phosphorus adsorbent of the present invention, the pH change is -1.0 or more and +1.0 or less (within ±1.0), preferably within ±0.8, more preferably within ±0.6. Most preferably, the phosphorus adsorbent has almost no pH change, that is, the pH change is within ±0.1. The method of measuring the pH of a phosphorus adsorbent in the present invention is as described later.

<Porous fiber>

The porous fiber of the present invention is a porous fiber in which a phosphorus adsorbent is supported, having a pH change of -1.0 or more and ±1.0 or less after being stirred for 4 hours in physiological saline.

In the present invention, the state in which the phosphorus adsorbent is supported in the porous fiber mainly means that the phosphorus adsorbent is physically mixed with the porous fiber, and is retained in the porous fiber while being attached to the inside of the porous fiber so that the function of the phosphorus adsorbent will not be impaired. However, the state is not necessarily limited to a state in which the phosphorus adsorbent is physically mixed with the porous fiber, and a state in which the phosphorus adsorbent is chemically bonded to the constituent molecules inside the porous fiber can also be regarded as the state in which the phosphorus adsorbent is supported in the porous fiber as long as the function of the phosphorus adsorbent is not impaired.

The porous fiber of the present invention has a pH change of -1.0 or more and +1.0 or less after being stirred for 4 hours in physiological saline. The pH change is preferably -0.8 or more and +0.8 or less (within ±0.8), more preferably within ±0.6. Most preferably, the porous fiber has almost no pH change, that is, the pH change is within ±0.1. As described above, in general, the pH balance in the living body is very important, and any imbalance of the pH may lead to various diseases. Since the porous fiber of the present invention causes a small change in the pH of physiological blood when being brought into contact with blood, the porous fiber has little influence on the pH of body fluids such as blood. Herein, a pH change in physiological saline is used as an index for determining whether or not the porous fiber is excellent in the pH balance in the living body. Specifically, in a simulation of 4-hour circulation in dialysis, the porous fiber supporting the phosphorus adsorbent therein is put in physiological saline, stirred at 200 rpm for 4 hours, and a change in the pH before and after the 4-hour stirring is measured. The method of measuring the pH of a porous fiber in the present invention is as described later.

The porous fiber of the present invention preferably has a phosphorus adsorption capability of 3.0 mg/g or more. The phosphorus adsorption capability is more preferably 3.5 mg/g or more, still more preferably 4.0 mg/g or more, especially preferably 7.0 mg/g or more, particularly preferably 15.0 mg/g or more. A phosphorus adsorption capability of the porous fiber of 3.0 mg/g or more easily produces a sufficient effect of adsorbing and removing phosphorus in an extracorporeal circulation column as a final form without the need to pack, in the column, a large amount of the porous fiber. As a result, it is easy to reduce the amount of blood taken out of the body for sufficient phosphorus removal. The method of measuring the phosphorus adsorption capability of a porous fiber in the present invention is as described later.

In the porous fiber of the present invention, it is preferable that the phosphorus adsorbent be a granule, and the granule have a solubility of 10 mg or less in 100 g of water at 20°C in a state of being supported in the porous fiber. The phosphorus adsorbent is preferably a granule, since homogeneity of the phosphorus adsorbent can be easily achieved, and the processing operation is facilitated. In addition, since the granule has a solubility of 10 mg or less in 100 g of water at 20°C in a state of being supported in the porous fiber, the amount of metal ions leaked out when the porous fiber comes into contact with the blood is reduced, so that the porous fiber can be favorably used. Herein, the solubility of the granule in 100 g of water at 20°C in a state of being supported in the porous fiber is measured by the method described later.

In the porous fiber of the present invention, it is preferable that the phosphorus adsorbent be a granule, and the granule have an average particle diameter more than 100 nm and 100 μm or less. The average particle diameter is preferably 100 μm or less, more preferably 80 μm or less, particularly preferably 50 μm or less, since the phosphorus adsorbent is preferably a granule that is as small as possible. An average particle diameter of 100 μm or less is preferable because the spinneret is hardly clogged during the spinning, and the spinning performance is hardly deteriorated. Moreover, as the average particle diameter is smaller, the specific surface area is larger, and the phosphorus adsorption efficiency is more easily improved. However, if the average particle diameter is smaller than the pore size of the porous fiber, the phosphorus adsorbent may pass through the pores and leak out into a liquid to be treated such as blood. Thus, the average particle diameter is preferably larger than the pore size, and preferably exceeds 100 nm. However, when the granule is an aggregate of primary particles, the size of the primary particles is not limited to the above-mentioned size.

The phosphorus adsorbent supported in the porous fiber of the present invention may be the above-mentioned phosphorus adsorbent of the present invention, but is not limited thereto. The phosphorus adsorbent is a compound having a phosphorus adsorption capability, that is, a compound with which a decrease in the phosphorus concentration from a phosphorus starting solution is observed in the measurement of the phosphorus adsorption capability described later.

In the porous fiber of the present invention, the phosphorus adsorbent preferably contains a salt of a rare earth element or a Group 4 oxide. The salt of a rare earth element is preferable because it has low solubility in water and a high phosphorus adsorption capability. In addition, the Group 4 oxide is preferable because it has low solubility in water and is small in the pH change.

Among salts of rare earth elements, a carbonate of a rare earth element is more preferable. The carbonate of a rare earth element is suitable for extracorporeal circulation, because it has high phosphorus adsorptivity but has low solubility, and is small in the pH change, so that the porous fiber hardly releases inorganic ions when coming into contact with blood.

In the porous fiber of the present invention, it is preferable that the phosphorus adsorbent contain a carbonate of a rare earth element, and the rare earth element be selected from the group consisting of lanthanum, cerium, praseodymium, and neodymium. In this case, the carbonate of a rare earth element is lanthanum carbonate, cerium carbonate, praseodymium carbonate, or neodymium carbonate. These carbonates of rare earth elements are preferable because they have low solubility in water and are small in the pH change in the living body as described above. Neodymium carbonate and cerium carbonate having a high phosphorus adsorption capability are more preferable.

The phosphorus adsorbent supported in the porous fiber of the present invention is not limited to these compounds, and any compound may be used as long as it is small in the pH change and is within the scope of to the present invention.

It is preferable that the phosphorus adsorbent supported in the porous fiber of the present invention be the above-mentioned phosphorus adsorbent of the present invention.

It is preferable that the porous fiber of the present invention be in the form of a porous fiber into which a phosphorus adsorbent mixed with a biocompatible polymer is kneaded. In this case, the lower limit of the percentage of the phosphorus adsorbent is preferably 5% by mass, more preferably 20% by mass, still more preferably 40% by mass relative to the polymer to be mixed. The upper limit of the percentage of the phosphorus adsorbent is preferably 80% by mass, more preferably 70% by mass, still more preferably 60% by mass. When the percentage of the phosphorus adsorbent is 5% by mass or more, more preferably 20% by mass or more, still more preferably 40% by mass or more, in a column containing the porous fiber, the amount of the porous fiber required for sufficient phosphorus adsorption can be easily reduced, which makes it easier to reduce the volume of the column and reduce the amount of blood taken out of the body. On the other hand, when the percentage of the phosphorus adsorbent is 80% by mass or less, more preferably 70% by mass or less, still more preferably 60% by mass or less, sufficient fiber strength of the porous fiber is easily obtained, and good spinning performance is easily obtained. The percentage of the phosphorus adsorbent can be calculated according to the following formula 1.

```
Formula 1: percentage (%): [mass of added phosphorus

adsorbent]/(mass of added phosphorus adsorbent + mass of raw

material polymer) × 100
```

The porous fiber of the present invention preferably has an average pore radius of 0.5 to 100 nm, and a specific surface area of pores of 10 $m^2$/g or more. The lower limit of the average pore radius (hereinafter also referred to as "pore size") is preferably 0.5 nm, more preferably 1.5 nm, particularly preferably 2.0 nm. On the other hand, the upper limit of the average pore radius is preferably 100 nm, more preferably 40 nm, particularly preferably 25 nm. When the lower limit of the average pore radius is as described above, a substance to be adsorbed easily enters the pores, so that the adsorption efficiency is hardly decreased. On the other hand, when the upper limit of the pore size is as described above, a substance to be adsorbed is likely to be adsorbed to the void portion, so that the adsorption efficiency may be improved in some cases. An optimum pore size lies within the above-mentioned pore size range depending on the size of the substance to be adsorbed and removed.

The average pore radius of the porous fiber is determined as a primary average pore radius according to differential scanning calorimetry (DSC) with a differential scanning calorimeter (DSC) through the measurement of the freezing point depression caused by capillary aggregation of water in the pores . The primary average pore radius of the pores is obtained in the following manner: an adsorbing material is rapidly cooled to -55°C and heated up to 5°C at a rate of 0.3°C/min, a measurement curve is obtained, and the primary average pore radius of the pores is calculated according to the following formula 2 with the peak top temperature of the curve being taken as the melting point.

$$\text{Formula 2: primary average pore radius [nm]} = (33.30 -$$

$$0.3181 \times \text{melting point depression amount [°C])/melting point}$$

$$\text{depression amount [°C]}$$

In the above-mentioned methods of measurement and calculation, reference is made to the description of Non-Patent Document 2.

The porous fiber of the present invention can have improved adsorption capability if it has a larger specific surface area of the pores for adsorbing a substance to be adsorbed. Therefore, the lower limit of the specific surface area of the pores is preferably 10 m$^2$/g, more preferably 20 m$^2$/g, still more preferably 30 m$^2$/g, particularly preferably 40 m$^2$/g, more particularly preferably 50 m$^2$/g. On the other hand, if the specific surface area of the pores is too large, the porous fiber is insufficient in mechanical strength. Therefore, the upper limit of the specific surface area of the pores is preferably 1000 m$^2$/g, more preferably 800 m$^2$/g, still more preferably 650 m$^2$/g, particularly preferably 500 m$^2$/g.

The specific surface area of the pores is measured by the DSC similarly to the method of measuring the average pore radius . The method of calculating the specific surface area of the pores is as described in Non-Patent Document 2.

The porous fiber of the present invention preferably has pores not only on the surface but also in the inside.

The porous fiber of the present invention preferably supports the phosphorus adsorbent not only on the surface but also in the inside in order to improve the efficiency of adsorbing phosphorus. In extracorporeal circulation, it is preferable to reduce the amount of blood taken out of a patient as much as possible for preventing the risk of blood pressure drop in the patient. For this purpose, it is preferable that the phosphorus adsorption capability per fiber volume be high. When the porous fiber supports the phosphorus adsorbent not only on the surface but also in the inside, the phosphorus adsorbent efficiently comes into contact with the targeted phosphorus-containing blood, so that the phosphorus adsorption capability per unit volume greatly increases, and it is possible to easily remove phosphorus contained in the blood efficiently. Therefore, a porous fiber in which the phosphorus adsorbent is supported, in other words, a porous fiber into which the adsorbent is kneaded is preferable. That is, it is preferable to make the phosphorus adsorbent present in the porous fiber with high dispersibility.

As a method for obtaining such a porous fiber, there is a method of adding a phosphorus adsorbent to a polymer solution prepared to have a predetermined concentration, and mixing the resultant mixture at a constant rotation speed. Alternatively, there is a method of putting a polymer raw material, a phosphorus adsorbent, and a solvent for dissolving the polymer raw material in one container from the beginning, and mixing the resultant mixture with a rotating rotor. Still alternatively, there is also a method of spraying and adding a phosphorus adsorbent to a polymer solution in which a polymer raw material is already dissolved at a predetermined concentration. The method is not limited to the above-mentioned methods as long as the phosphorus adsorbent can be uniformly dispersed in the polymer. In the case of a polymer that gelates at room temperature, it is preferable to heat the polymer in order to improve the dissolution efficiency of the polymer.

<Material of porous fiber>

The material of the porous fiber in the present invention is not particularly limited, but organic materials are suitably used from the viewpoint of ease of molding processing and cost. More specifically, polymethyl methacrylate (hereinafter referred to as PMMA), polyacrylonitrile (hereinafter referred to as PAN), polysulfone, polyether sulfone, polyaryl ether sulfone, polypropylene, polystyrene, polycarbonate, cellulose, cellulose triacetate, an ethylene-vinyl alcohol copolymer, and the like are suitably used. Above all, the porous fiber preferably contains a material that is an amorphous polymer and has a property of adsorbing proteins, such as PMMA and PAN. PMMA and PAN are representative examples of a fiber having a uniform structure in the thickness direction, and are preferable since they easily gives a homogeneous structure having a sharp pore size distribution. In particular, PMMA is preferable, because PMMA is excellent in molding processability and low in cost, and has high transparency, so that the internal state of the porous fiber containing PMMA can be relatively easily observed, and the state of fouling of the porous fiber can be easily evaluated.

<Thread shape of porous fiber>

Further, when the porous fiber has a hollow fiber shape or a solid fiber shape, a sufficient area for adsorption is easily secured using a porous material suitable for adsorption in the film thickness part or inside the threads, respectively, and consequently, it becomes easy to efficiently adsorb and remove the adsorption target substance contained in the blood. Of these, a solid fiber shape is particularly preferable. This is because in the case of a hollow fiber, if the pressure loss differs between the inside and the outside of the hollow fiber, the flow rate of the liquid to be treated may vary between the inside and the outside of the hollow fiber, resulting in concern about a decrease in the adsorption efficiency of the column. In addition, in order to equalize the pressure loss between the inside and the outside of a hollow fiber, great limitations are imposed on the inner diameter of the hollow fiber and the column packing rate. Furthermore, in the case where blood is allowed to flow through a column packed with a hollow fiber, concern is generated about the tendency of formation of blood clots because the hollow portion of the hollow fiber is a fixed and closed environment in comparison with the environment outside the hollow fiber in the column (the space outside the hollow fiber deforms as the threads move in the column).

Further, in the case of a solid fiber, the cross-sectional shape of the fiber is not necessarily limited to a perfect circle, and solid fibers having elliptical holes, those having rectangular and triangular cross sections, and those having modified cross-sectional shapes can also be suitably used depending on the circumstance.

<Method of measuring fiber diameter>

The porous fiber of the present invention preferably has a diameter of 50 to 1000 $\mu$m. The fiber diameter is preferably 50 $\mu$m or more, more preferably 100 $\mu$m or more, still more preferably 150 $\mu$m or more. The fiber diameter is preferably 1000 $\mu$m or less, more preferably 800 $\mu$m or less, still more preferably 500 $\mu$m or less. When the fiber diameter is 50 $\mu$m or more, more preferably 100 $\mu$m or more, still more preferably 150 $\mu$m or more, the fiber strength hardly decreases even if the percentage of the particles is increased, and the productivity tends to be improved. On the other hand, when the fiber diameter is 1000 $\mu$m or less, more preferably 800 $\mu$m or less, still more preferably 500 $\mu$m or less, the packing rate of the adsorbing fiber packed in the column easily increases, and the adsorption capability is easily improved.

The method of measuring the fiber diameter is as follows. More specifically, 50 threads arbitrarily selected from threads (fiber) packed in a column are extracted and washed. After washing, the cleaning liquid is completely replaced with pure water. The threads are sandwiched between a slide glass and a cover glass. The outer diameters (diameters of the outermost circumference) of one thread at arbitrary two positions are measured with a projector (V-10A manufactured by NIKON CORPORATION, for example) . The average of the outer diameters at the two positions is calculated, and the value is rounded off to the nearest whole number. If the number of packed threads is less than 50, all the threads are measured, and the average thereof is similarly calculated. If the cross-sectional shape of the porous fiber is not a perfect circle, the diameter of the fiber is calculated according to the formula $(a \times b)^{0.5}$ wherein the diameter of a circle inscribed in the cross-sectional shape of the porous fiber is "a", and the diameter of a circle circumscribed about the cross-sectional shape of the porous fiber is "b".

<Phosphorus adsorption column>

Another aspect of the phosphorus adsorption column of the present invention is a phosphorus adsorption column containing the porous fiber of the present invention incorporated therein.

In the following, an example of producing the phosphorus adsorption column (hereinafter sometimes simply referred to as "column") of the present invention using a porous fiber having a solid fiber shape will be described, but the present invention is not limited thereto.

<Spinning method>

A polymer serving as a raw material of the porous fiber is dissolved in an appropriate solvent, and then a predetermined amount of particles of a selected phosphorus adsorbent are added to the resultant solution to prepare a spinning stock solution. The solvent is appropriately selected according to the polymer to be dissolved therein. In general, dimethylformamide, dimethylsulfoxide, hexanone, xylene, tetralin, cyclohexanone, carbon tetrachloride and the like are used. For example, when PMMA is used as a polymer, dimethylsulfoxide (DMSO) is preferably used.

If the viscosity of the spinning stock solution is too low, the stock solution has high fluidity, and it may be difficult to maintain the desired shape. Therefore, the lower limit of the viscosity of the stock solution at the temperature of the spinneret part is preferably 10 poise, more preferably 90 poise, still more preferably 400 poise, particularly preferably 800 poise. On the other hand, if the viscosity of the spinning stock solution is too high, the discharge stability tends to be low due to an increase in the pressure loss at the time of discharge of the stock solution, or mixing of the stock solution may be difficult. Therefore, the upper limit of the viscosity of the stock solution at the temperature of the spinneret part is preferably 100000 poise, more preferably 50000 poise.

The spinning stock solution is discharged from a spinneret having a circular stock solution discharge port, or when it is intended to make a fiber having a modified cross-sectional shape, the spinning stock solution is discharged from a spinneret having a stock solution discharge port having a shape conforming to the cross-sectional shape. Then, the spinning stock solution is coagulated in a coagulation bath into a solid fiber shape. The coagulation bath is usually made from a coagulant such as water or alcohol, or a mixture of a solvent that constitutes the spinning stock solution and a coagulant. The porosity of the porous fiber can be varied by controlling the temperature of the coagulation bath. Since the porosity can be influenced by the type of the spinning stock solution and other factors, the temperature of the coagulation bath is appropriately selected. In general, the porosity can be increased by raising the temperature of the coagulation bath. Although the exact mechanism is not clear, it is considered that in a competition reaction of removal of the solvent from the stock solution and coagulation shrinkage, the solvent is rapidly removed in a high-temperature bath, and the stock solution is coagulated and fixed before shrinking. However, if the coagulation bath temperature is too high, a porous fiber having too large a pore size is obtained. Therefore, for example, when a solid fiber containing PMMA as a base material is to be obtained and a gas is introduced into the inner tube of the spinneret, the coagulation bath temperature is preferably 39°C or higher, more preferably 42°C or higher. On the other hand, the coagulation bath temperature is preferably 50°C or lower, more preferably 46°C or lower.

Then, the solid fiber obtained by coagulation is passed through a step of washing off the solvent attached to the solid fiber. Although the means for washing the solid fiber is not particularly limited, it is preferable to employ a method of passing the solid fiber through a multi-stage water bath (referred to as "water washing bath"). The temperature of the water in the water washing bath may be determined according to the properties of the polymer making up the solid fiber. For example, in the case of a solid fiber containing PMMA, water at 30 to 50°C is used.

Further, in order to maintain the pore size of the solid fiber after having passed through the water washing bath, a step of applying a moisturizing component may be added. The moisturizing component as used herein is a component capable of maintaining the moisture in the solid fiber, or a component capable of preventing reduction of the moisture in the solid fiber in the air. Representative examples of the moisturizing component include glycerin and an aqueous solution of glycerin.

After completion of water washing and application of a moisturizing component, it is also possible to pass the solid fiber through a step of passing the solid fiber through a bath filled with an aqueous solution of a heated moisturizing component (referred to as "heat treatment bath") in order to increase the dimensional stability of the highly shrinkable solid fiber. The solid fiber passed through the heat treatment bath filled with an aqueous solution of a heated moisturizing component shrinks by the action of heat, and becomes difficult to shrink in the subsequent process, so that the fiber structure can be stabilized. The heat treatment temperature in this process varies depending on the material of the porous fiber. In the case of a fiber containing PMMA, the heat treatment temperature is preferably 75°C or higher, more preferably 82°C or higher. The heat treatment temperature is preferably 90°C or lower, more preferably 86°C or lower.

<Casing shape>

The solid fiber produced in this manner is incorporated in a casing to form a phosphorus adsorption column.

The shape of the casing may be a shape that is open at both ends, for example, polygonal tubes such as a square tube and a hexagonal tube, and a cylinder. Among them, a cylinder, particularly a cylinder having a perfectly circular cross section is preferable. This is because a casing that does not have a corner makes it easy to suppress the retention of the blood as a liquid to be treated at the corner. In addition, a casing that is open at both ends makes it easy to prevent the flow of the liquid to be treated from becoming a turbulent flow, and to suppress the pressure loss to the minimum.

The casing is preferably an instrument made of plastics, metal, or the like. In the former case, the casing is produced by injection-molding the material with a mold, or by cutting the material. In the latter case, the instrument is produced by cutting the material. Among the materials, plastics are preferably used from the viewpoint of cost, moldability, weight, and blood compatibility.

In the case of plastics, for example, a thermoplastic resin excellent in mechanical strength and thermal stability is used. Specific examples of the thermoplastic resin include polycarbonate resins, polyvinyl alcohol resins, cellulose resins, polyester resins, polyarylate resins, polyimide resins, cyclic polyolefin resins, polysulfone resins, polyethersulfone resins, polyolefin resins, polystyrene resins, polyvinyl alcohol resins, and mixtures thereof. Among them, polystyrene, polycarbonate, and derivatives thereof are preferable in terms of moldability, transparency, and radiation resistance required of the casing. A resin excellent in transparency is convenient for securing safety since the internal state of the casing can be checked during blood perfusion, and a resin excellent in radiation resistance is preferable for radiation irradiation at sterilization.

The casing length of the phosphorus adsorption column of the present invention is preferably 1 cm or more and 500 cm or less, more preferably 3 cm or more and 50 cm or less. Herein, the casing length is the length of a cylindrical casing in the axial direction before a partition wall is provided or a cap is mounted. When the casing length of the column is 500 cm or less, more preferably 50 cm or less, it is believed that insertion of the porous fiber into the column tends to be facilitated, and it tends to become easy to handle the phosphorus adsorption column in practical use. When the casing length is 1 cm or more, more preferably 3 cm or more, the length tends to work advantageously, for example, in the case of forming a partition wall, and the handling property of a column including the casing is likely to be improved.

The shape of the porous fiber when incorporated in the column is preferably a straight shape, and it is preferable to insert a straight fiber in parallel to the longitudinal direction of the column case. Since a straight-shaped porous fiber easily secures the flow path of a liquid to be treated, it is easy to evenly distribute the liquid to be treated in the column, and to suppress the flow path resistance. The straight-shaped porous fiber is also advantageous in terms of an increase in the pressure loss due to, for example, attachment of a solute in the liquid to be treated. Therefore, even when the liquid to be treated is highly viscous blood, the risk of coagulation and the like in the casing can be easily suppressed. Moreover, the porous fiber can be processed into a knitted fabric, a woven fabric, a nonwoven fabric or the like. However, since large tension is applied to the threads during processing, the porous fiber has restrictions such as impossibility of increasing the porosity of the porous fiber. In addition, processing the porous fiber may increase the number of steps and increase the cost.

The number of threads of the porous fiber incorporated in the phosphorus adsorption column of the present invention is preferably about 1,000 to 500,000.

<Packing rate>

The upper limit of the packing rate of an adsorbent material in the column casing is preferably 70%, more preferably 63%. The lower limit of the packing rate of the adsorbent material is preferably 13%, more preferably 30%, particularly preferably 45%. Setting the packing rate of the adsorbent material to 13% or more reduces the amount of blood required for blood purification, so that it is easy to reduce the burden on patients. A packing rate of the adsorbent material of 70% or less tends to improve the air release property. Further, the working efficiency tends to be improved since it becomes easier to pack the adsorbent material. The packing rate as used herein is the rate of the volume of the adsorbent material in the volume of the casing that has an inlet into which the unpurified blood flows and an outlet from which the purified blood is discharged, and the volume of the casing does not include that of the header part.

The packing rate is the ratio between the casing volume (Vc) and the fiber volume (Vf). The casing volume (Vc) is calculated from the cross-sectional area and the length of the casing, and the fiber volume (Vf) is calculated from the cross-sectional area of the fiber, the casing length, and the number of threads of the fiber. The packing rate is calculated as follows.

```
Vc = cross-sectional area of casing body × effective

length of casing
```

```
Vf = cross-sectional area of fiber × number of threads

of fiber × effective length of casing
```

```
Packing rate = Vf/Vc × 100 (%)
```

If the casing is tapered, the cross-sectional area of the casing body means the cross-sectional area at the center of the casing.

Herein, the casing volume Vc does not include the volume of a member that does not contain a fiber, for example, members serving as inlet/outlet ports of a liquid to be treated, such as a header and a header cap. As for the fiber volume Vf, in the case where a spacer fiber or the like for preventing close contact between threads in the casing is used, the fiber volume includes the volume of the spacer fiber or the like. The effective length of the fiber is a length obtained by subtracting the length of the partition wall from the casing length. The upper limit of the effective length of the fiber is preferably 5000 mm, more preferably 500 mm, particularly preferably 210 mm from the viewpoint of preventing curving of the fiber, and ease of reduction of the pressure loss in a column including the casing. The lower limit of the effective length of the fiber is preferably 5 mm, more preferably 20 mm, particularly preferably 30 mm from the viewpoint of reducing the amount of threads to be discarded, for example, when cutting extra threads sticking out of the column in order to equalize the length of the threads, improving the productivity, and facilitating handling of a fiber bundle. In the case of crimped threads, the method of measuring the effective length of the fiber is a method of measuring the length of straight threads obtained by stretching both ends of the crimped threads. Specifically, one end of a fiber taken out of the column is fixed with a tape or the like and vertically lowered, a weight of about 8 g per cross-sectional area $(mm^2)$ of the threads is applied to the other end of the fiber, and the total length of the fiber in a straight state is rapidly measured. This measurement is carried out for 30 threads of the fiber arbitrarily selected in the column, and the average of the 30 threads is calculated in mm and rounded off to the nearest whole number.

<Sterilization method>

When the column is used in medical devices and the like, the column is preferably used after disinfection or sterilization. Examples of disinfection and sterilization methods include various disinfection and sterilization methods, such as high-pressure steam sterilization, gamma-ray sterilization, ethylene oxide gas sterilization, drug sterilization, and UV sterilization. Among these methods, gamma-ray sterilization, high-pressure steam sterilization, and ethylene oxide gas sterilization are preferable because they have small influence on the sterilization efficiency and materials.

<Phosphorus adsorption capability of column>

The phosphorus adsorption capability of the column produced as described above can also be measured. A detailed measurement method will be described later in the examples . The phosphorus adsorption capability of the column is preferably 3.0 mg/g or more, more preferably 4.0 mg/g or more, still more preferably 5.0 mg/g or more, especially preferably 10.0 mg/g or more, particularly preferably 15.0 mg/g or more.

<Usage form>

The phosphorus adsorption column of the present invention is used in various applications, and can be used in applications such as water treatment, purification, and blood purification. In the blood purification application, the treatment method may be either of a method of directly perfusing whole blood, and a method of separating plasma from blood and then passing the plasma through a column. The phosphorus adsorption column of the present invention can be used in either of the methods.

When the phosphorus adsorption column is used as a blood purifier, it is preferable to employ a method of incorporating the phosphorus adsorption column in an extracorporeal circulation circuit and performing adsorption and removal on-line from the viewpoint of the treatment amount per single operation and convenience of operation. In this case, the phosphorus adsorption column of the present invention may be used singly, or may be used in connection with an artificial kidney in series during dialysis or the like. Such a method makes it possible to perform the dialysis simultaneously with removal of substances that are insufficiently removed only with an artificial kidney. In particular, the phosphorus adsorption column of the present invention can complement the function of an artificial kidney by adsorbing and removing phosphorus that is insufficiently removed with an artificial kidney.

When being used simultaneously with an artificial kidney, the phosphorus adsorption column may be connected in front of an artificial kidney or at the back of an artificial kidney in a circuit. An advantage of connecting the phosphorus adsorption column in front of an artificial kidney is that the phosphorus adsorption column easily exerts the original performance because it is hardly influenced by dialysis with the artificial kidney. On the other hand, an advantage of connecting the phosphorus adsorption column at the back of an artificial kidney is that the solute concentration in the blood or the like to be treated is high because water has already been removed from the blood in the artificial kidney, and an increase in the efficiency of adsorption and removal of phosphorus can be expected.

EXAMPLES

[Examples 1 to 5 and Comparative Examples 1 to 3]

(1) Evaluation of phosphorus adsorption capability

Bovine plasma was obtained by centrifugation from bovine blood to which disodium ethylenediaminetetraacetate had been added. The total protein (TP) of the bovine plasma was adjusted to 6.5 $\pm$ 0.5 g/dL. Bovine plasma within 5 days after blood collection was used.

To 400 mL of the bovine plasma, 31.4 mg of sodium monohydrogen phosphate ($Na_2HPO_4$) and 13.8 mg of potassium dihydrogen phosphate ($KH_2PO_4$) were dissolved to give a phosphorus starting solution. The phosphorus concentration of the phosphorus starting solution was defined as Cs (mg/dL).

To 0.01 g of each of the phosphorus adsorbents shown in Table 1, 50 mL of the phosphorus starting solution was added, and the resultant mixture was shaken at 37°C for 4 hours for reaction. After the reaction for 4 hours, the reaction liquid was centrifuged at 9000 rpm for 5 minutes, and the supernatant was collected. The supernatant was sent to Nagahama Life Science Laboratory of Oriental Yeast Co., ltd. The phosphorus concentration in the supernatant was measured as the final concentration Ce (mg/dL). The phosphorus starting solution was also sent to Nagahama Life Science Laboratory for measurement. Then, the phosphorus adsorption capability per 1 g of the adsorbent was calculated according to the following formula 3. The phosphorus adsorption capability obtained from this formula is a value in the case where the adsorption is not saturated.

$$\text{Formula 3: phosphorus adsorption capability [mg/g]} = [(Cs - Ce) \times 0.5 \ (dL)]/0.01 \ (g)$$

The method of measuring the phosphorus adsorption capability of a porous fiber is the same as the method of measuring the phosphorus adsorption capability of a phosphorus adsorbent except that 0.02 g of a porous fiber supporting a phosphorus adsorbent therein was used instead of 0.01 g of the phosphorus adsorbent. In the calculation of the phosphorus adsorption capability, the following formula 4 was used.

$$\text{Formula 4: phosphorus adsorption capability of porous fiber [mg/g]} = [(Cs \ (mg/dL) - Ce \ (mg/dL)) \times 0.5 \ (dL)]/\text{mass of porous fiber (g)}$$

(2) Average particle diameter of phosphorus adsorbent

The average particle diameter of a phosphorus adsorbent was measured with MT3300 manufactured by NIKKISO CO., LTD. by a laser diffraction/scattering method. Using nonspherical particles, the particle diameter and the frequency (%) were plotted on the horizontal axis and the vertical axis, respectively, and the number average particle diameter was used as the average particle diameter.

(3) pH measurement

As the pH measurement method, the most widely used measurement method, that is, a glass electrode method was used. This is a method of measuring the pH of a target solution by obtaining the voltage (potential difference) generated between two electrodes, that is, a glass electrode and a reference electrode. Specifically, a compact pH meter LAQUAtwin manufactured by HORIBA, Ltd. or the like can be used.

The pH of a phosphorus adsorbent was measured with a compact pH meter LAQUAtwin manufactured by HORIBA, Ltd. First, the pH scale was calibrated with known standard buffers (pH 4.01 and 6.86). The pH of physiological saline was measured and regarded as pH (start). A phosphorus adsorbent (0.1 g) was weighed, added to 10 mL of physiological saline, and stirred at room temperature for 4 hours. After stirring, 1 mL of the physiological saline solution to which the phosphorus adsorbent had been added was sampled, and centrifuged at 9000 rpm for 5 minutes. To a measurement chamber, 500 $\mu$L of the supernatant was added, and the pH was measured as pH (4H). The pH change was determined according to the formula 5.

In the case of measuring the pH of a porous fiber, the pH was measured in the same manner as in the case of measuring a phosphorus adsorbent except that 0.2 g of a porous fiber was weighed as a sample.

$$\text{Formula 5: pH change} = pH \ (4H) - pH \ (start)$$

(4) Solubility measurement

Water (100 g) adjusted to 20°C in a thermostatic bath and a rotor were put in a flask. To the flask, 100 mg of a phosphorus adsorbent was charged, and the resultant mixture was stirred for 4 hours or more. Then, the mixture was centrifuged at 12000 rpm for 20 minutes to separate the mixture into particles of the phosphorus adsorbent and the solution. The supernatant was sampled, and the particle ion concentration of the dissolved phosphorus adsorbent was measured. When the adsorbent was in the form of a granule, the particle ion concentration was measured by inductively coupled plasma-mass spectrometry (ICP-MS). The particle mass was calculated from the measured particle ion concentration, and the mass of the dissolved particles per 100 g of the supernatant was taken as the solubility.

In the case of a porous fiber, the same evaluation as above was carried out except that 200 mg of a porous fiber was added to 100 g of water.

[Example 6]

First, a 21% by mass PMMA stock solution was prepared. 31.7 parts by mass of syndiotactic-PMMA having a mass average molecular weight of 400,000 (syn-PMMA, manufactured by MITSUBISHI RAYON CO., LTD., "Dianal" BR-85), 31.7 parts by mass of syn-PMMA having a mass average molecular weight of 1,400,000 (manufactured by Sumitomo Chemical Company, Limited, "Sumipex" AK-150), 16.7 parts by mass of isotactic-PMMA having a mass average molecular weight of 500,000 (iso-PMMA, manufactured by TORAY INDUSTRIES, INC.), and 20 parts by mass of PMMA copolymer having a molecular weight of 300,000 and containing 1.5 mol% of sodium p-styrenesulfonate (manufactured by TORAY INDUSTRIES, INC.,),was mixed with 376 parts by mass of dimethylsulfoxide (DMSO), and the resultant mixture was stirred at 110°C for 8 hours to prepare a spinning stock solution. The viscosity of the obtained spinning stock solution at 110°C was 1240 poise.

Then, 1 g of titanium oxide particles (Example 5) as a phosphorus adsorbent were added to 50 g of the obtained spinning stock solution to prepare a titanium oxide/PMMA stock solution. The titanium oxide/PMMA stock solution was stirred at 100 rpm for uniform mixing while being heated at 100°C. The stock solution was added to a 10-mL syringe, and discharged with a syringe pump into a coagulation liquid maintained at a temperature of 45°C at a discharge linear velocity of 6 m/min, and then the formed fiber was automatically wound at 100 rpm/min. The fiber was thoroughly washed with pure water. A solid porous fiber containing 8.7% by mass of the phosphorus adsorbent and having an outer diameter of the cross section of 200 $\mu$m was obtained.

The phosphorus adsorption capability of the porous fiber obtained as described above was evaluated. To 50 mL of the phosphorus starting solution prepared in (1) Evaluation of phosphorus adsorption capability, 1 g of the porous fiber (containing 0.087 g of a phosphorus adsorbent) was added, and the resultant mixture was subjected to an adsorption reaction for 4 hours while being shaken at 37°C. During the shaking, the reaction liquid was sampled at predetermined time intervals, the sampled liquid was centrifuged at 9000 rpm for 5 minutes, the supernatant was collected, and the phosphorus concentration in the supernatant was determined. Meanwhile, the fiber after 4 hours of the reaction was washed with physiological saline, then further washed with water, and dried in an oven at 50°C overnight. After it was confirmed that the mass of the porous fiber had not been changed, the value obtained by weighing the porous fiber again was taken as the mass of the porous fiber. The phosphorus adsorption capability of the porous fiber was calculated according to the above formula 4. The results are shown in Table 2.

[Example 7]

Titanium oxide particles (4 g) as a phosphorus adsorbent were added to 50 g of the 21% by mass PMMA stock solution prepared in Example 6 to prepare a titanium oxide/PMMA stock solution. Then, the titanium oxide/PMMA stock solution was spun in the same manner as in Example 6. A solid porous fiber containing 27.6% by mass of the phosphorus adsorbent and having an outer diameter of the cross section of 350 $\mu$m was obtained.

The phosphorus adsorption capability of the porous fiber was evaluated. Similarly to Comparative Example 3, to 50 mL of the phosphorus starting solution prepared in (1) Evaluation of phosphorus adsorption capability shown in the measurement example, 1 g of the porous fiber (containing 0.275 g of a phosphorus adsorbent) was added. The phosphorus concentration and the mass of the porous fiber were determined in the same manner as in Example 6, and the phosphorus adsorption capability of the porous fiber was calculated according to the formula 4. The results are shown in Table 2.

[Example 8]

To 23.81 g of the 21% by mass PMMA stock solution prepared in Example 6, 26.19 g of DMSO was added to give 50 g of a polymer solution containing 10% by mass of PMMA. Titanium oxide particles (5 g) as a phosphorus adsorbent were added to 50 g of the 10% by mass PMMA stock solution, and the resultant mixture was heated to 100°C and uniformly mixed while being stirred at a rotation speed of 100 rpm to prepare a titanium oxide/PMMA stock solution. Then, the titanium oxide/PMMA stock solution was spun in the same manner as in Example 6. A solid porous fiber containing 50% by mass of the phosphorus adsorbent and having an outer diameter of the cross section of 250 $\mu$m was obtained.

To 0.1 g of the porous fiber (containing 0.05 g of a phosphorus adsorbent) obtained as described above, 50 mL of the phosphorus starting solution prepared in (1) Evaluation of phosphorus adsorption capability was added. The phosphorus concentration and the mass of the porous fiber were determined in the same manner as in Example 6, and the phosphorus adsorption capability of the porous fiber was calculated according to the formula 4.

[Example 9]

To 23.81 g of the 21% by mass PMMA stock solution prepared in Example 6, 26.19 g of DMSO was added to give 50 g of a polymer solution having a PMMA concentration of 10% by mass. Lanthanum carbonate particles (5 g) as a phosphorus adsorbent were added to 50 g of the 10% by mass PMMA stock solution, and the resultant mixture was heated to 100°C and uniformly mixed while being stirred at a rotation speed of 100 rpm to prepare a lanthanum carbonate/PMMA stock solution. Then, the lanthanum carbonate/PMMA stock solution was spun in the same manner as in Comparative Example 3. A solid porous fiber containing 50% by mass of the phosphorus adsorbent and having an outer diameter of the cross section of 170 $\mu$m was obtained.

To 0.1 g of the porous fiber (containing 0.05 g of a phosphorus adsorbent) obtained as described above, 50 mL of the phosphorus starting solution prepared in (1) Evaluation of phosphorus adsorption capability was added. The phosphorus concentration and the mass of the porous fiber were determined in the same manner as in Example 6, and the phosphorus adsorption capability of the porous fiber was calculated according to the formula 4.

[Comparative Example 4]

The stock solution was spun in the same manner as in Example 6 except that no particles were added to give a solid PMMA fiber having an outer diameter of the cross section of 138 $\mu$m. The obtained PMMA fiber was evaluated for the phosphorus adsorption capability in the same manner as in Example 6.

[Example 10 and Example 11]

<Production of column>

A plurality of threads of the porous fiber obtained in Example 8 or Example 9 were packed in a polycarbonate cylindrical casing having an inner diameter of 10 mm and an axial length of 12.2 mm.

More specifically, in Example 10, a total of 540 threads, which were obtained by cutting the threads having a diameter of 270 $\mu$m obtained in Example 9 into a length of 11 mm, were packed in the casing to form a column having a packing rate of 35.5%. Meanwhile, in Example 11, a total of 760 threads, which were obtained by cutting the threads having a diameter of 170 $\mu$m obtained in Example 8 into a length of 11 mm, were packed in the casing to form a column having a packing rate of 19.8%. Then, to the inlet and outlet for the liquid to be treated provided on both the end faces of each of these columns, a polypropylene mesh filter having an opening equivalent diameter of 84 $\mu$m and an opening ratio of 36% cut to have a diameter equivalent to the inner diameter of the casing was attached. Finally, a cap called a header having an inlet and an outlet for the liquid to be treated was attached to each end of the casing.

<Measurement of phosphorus adsorption capability of column>

As the adsorption capability evaluation, the phosphorus adsorption capability of the column was measured. Bovine plasma was obtained in the same manner as in (1) Evaluation of phosphorus adsorption capability of Examples 1 to 5 and Comparative Examples 1 to 3. The total protein of the bovine plasma was adjusted to 6.5 ± 0.5 g/dL. Bovine plasma within 5 days after blood collection was used. To 100 mL of the bovine plasma, 7.85 mg of sodium monohydrogen phosphate ($Na_2HPO_4$) and 3.45 mg of potassium dihydrogen phosphate ($KH_2PO_4$) were dissolved to give a liquid to be treated simulating the blood of a hyperphosphatemia patient.

A silicone tube was attached to the inlet and outlet of the column, and both the inlet and the outlet were immersed in the liquid to be treated to form a circulation system. The liquid to be treated was allowed to flow at a flow rate of 1 mL/min, and after passage through the column from the column inlet, the purified liquid from the outlet was returned to the liquid to be treated. The liquid to be treated and the purified liquid from the outlet were sampled, and the phosphorus concentration in the samples was measured. After circulation, the threads were washed with physiological saline and water, and then dried in an oven at 60°C overnight. After it was confirmed that the weight of the threads had not been changed, the weight was taken as the dry weight.

[Table 1]

| | Phosphorus adsorbent | Phosphorus adsorption capability [mg/g] | Solubility [mg/100 g$H_2O$] | Average particle diameter [$\mu$m] | pH change (4H) |
|---|---|---|---|---|---|
| Example 1 | Lanthanum carbonate | 15.6 | <1 | 0.7 | +0.5 |

(continued)

|  | Phosphorus adsorbent | Phosphorus adsorption capability [mg/g] | Solubility [mg/100 gH$_2$O] | Average particle diameter [μm] | pH change (4H) |
|---|---|---|---|---|---|
| Example 2 | Neodymium carbonate | 132.5 | <1 | 0.7 | +0.4 |
| Example 3 | Praseodymium carbonate | 17.0 | <1 | 1.1 | +0.1 |
| Example 4 | Cerium carbonate | 79.5 | <1 | 11.6 | +0.2 |
| Example 5 | Titanium oxide | 17.9 | <1 | 10.7 | +0.6 |
| Comparative Example 1 | Cerium hydroxide | 8.8 | <1 | 1.0 | -2.0 |
| Comparative Example 2 | Lanthanum hydroxide | 110.6 | <1 | 0.5 | +1.8 |
| Comparative Example 3 | Lanthanum oxide | 141.0 | <1 | 0.5 | +3.8 |

[Table 2]

|  | Porous fiber | Amount of added particles (% by mass) | Phosphorus adsorption capability [mg/g] | Fiber diameter (μm) | Pore size (nm) | Specific surface area (m$^2$/g) |
|---|---|---|---|---|---|---|
| Example 6 | Titanium oxide fiber | 8.75 | 1.0 | 200 | 11 | 306 |
| Example 7 | Titanium oxide fiber | 27.5 | 3.5 | 350 | ND | ND |
| Example 8 | Titanium oxide fiber | 50 | 7.2 | 270 | 15 | 66 |
| Example 9 | Lanthanum carbonate fiber | 50 | 19.2 | 170 | ND | ND |
| Comparative Example 4 | PMMA fiber | 0 | 0 | 138 | ND | ND |

[Table 3]

|  | Porous fiber | Amount of added particles (% by mass) | Phosphorus adsorption capability [mg/g] | Fiber diameter (μm) | Pore size (nm) | Specific surface area (m$^2$/g) |
|---|---|---|---|---|---|---|
| Example 10 | Lanthanum carbonate fiber | 50 | 15.9 | 170 | ND | ND |
| Example 11 | Titanium oxide fiber | 50 | 6.25 | 270 | 15 | 66 |

The following numbered clauses, describing aspects of our proposals, are part of the description:

1. A phosphorus adsorbent for use in phosphorus adsorption in extracorporeal circulation, comprising a carbonate of a rare earth element or a Group 4 oxide, and being a granule having a solubility of 10 mg or less in 100 g of water at 20°C.

2. The phosphorus adsorbent according to clause 1, wherein the rare earth element is selected from the group consisting of lanthanum, cerium, praseodymium, and neodymium.

3. The phosphorus adsorbent according to clause 1 or 2, being a granule having an average particle diameter more than 100 nm and 100 $\mu$m or less.

4. The phosphorus adsorbent according to any one of clauses 1 to 3, having a phosphorus adsorption capability of 4.0 mg/g or more.

5. A porous fiber in which a phosphorus adsorbent is supported, having a pH change of -1.0 or more and +1.0 or less after being stirred for 4 hours in physiological saline.

6. The porous fiber according to clause 5, having a phosphorus adsorption capability of 3.0 mg/g or more.

7. The porous fiber according to clause 5 or 6, wherein the phosphorus adsorbent is a granule, and the granule has a solubility of 10 mg or less in 100 g of water at 20°C in a state of being supported in the porous fiber.

8. The porous fiber according to any one of clauses 5 to 7, wherein the phosphorus adsorbent is a granule, and the granule has an average particle diameter more than 100 nm and 100 $\mu$m or less.

9. The porous fiber according to any one of clauses 5 to 8, having a diameter of 50 to 1000 $\mu$m.

10. The porous fiber according to any one of clauses 5 to 9, having an average pore radius of 0.5 to 100 nm, and a specific surface area of pores of 10 $m^2$/g or more.

11. The porous fiber according to any one of clauses 5 to 10, wherein the phosphorus adsorbent contains a salt of a rare earth element or a Group 4 oxide.

12. The porous fiber according to clause 11, wherein the phosphorus adsorbent contains a carbonate of a rare earth element, and the rare earth element is selected from the group consisting of lanthanum, cerium, praseodymium, and neodymium.

13. A phosphorus adsorption column, comprising the phosphorus adsorbent according to any one of clauses 1 to 4 incorporated therein.

14. A phosphorus adsorption column, comprising the porous fiber according to any one of clauses 5 to 12 incorporated therein.


**Claims**

1. A phosphorus adsorbent for use in phosphorus adsorption in extracorporeal circulation, comprising a carbonate of neodymium, and being a granule having a solubility of 10 mg or less in 100 g of water at 20°C.

2. The phosphorus adsorbent according to claim 1, being a granule having an average particle diameter more than 100 nm and 100 $\mu$m or less.

3. The phosphorus adsorbent according to claim 1 or 2, having a phosphorus adsorption capability of 70.0 mg/g or more.

4. The phosphorus adsorbent according to any one of claims 1 to 3, having a pH change of -1.0 or more and +1.0 or less after being stirred for 4 hours in physiological saline.

5. A porous fiber in which the phosphorus adsorbent according to any one of claims 1 to 4 is supported.

6. A phosphorus adsorption column, comprising the phosphorus adsorbent according to any one of claims 1 to 4 incorporated therein.

Europäisches
Patentamt

European
Patent Office

Office européen
des brevets

**EUROPEAN SEARCH REPORT**

Application Number

EP 20 19 7505

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | CN 103 011 240 A (GANZHOU QIANDONG RARE EARTH GROUP CO LTD) 3 April 2013 (2013-04-03) * paragraph [0033] * * paragraph [0037]; example 2 * | 1-4 | INV. A61M1/36 B01D15/08 B01J20/02 B01J20/06 |
| X | US 2002/017635 A1 (WATAYA KAZUHIRO [JP] ET AL) 14 February 2002 (2002-02-14) * claims 3, 4 * | 1-4 | B01J20/28 B01J20/32 C01F17/00 C01G23/047 B01D15/12 C01F17/247 |
| A | US 2007/149405 A1 (SPITLER TIMOTHY M [US]) 28 June 2007 (2007-06-28) * abstract * * paragraphs [0008], [0009] * * paragraph [0030]; example II * * claims 10, 11 * | 1-6 | |

TECHNICAL FIELDS SEARCHED (IPC)

A61M
B01D
B01J
C02F
C01G
C01F

The present search report has been drawn up for all claims

| Place of search | Date of completion of the search | Examiner |
|---|---|---|
| The Hague | 21 December 2020 | Klemps, Christian |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

## ANNEX TO THE EUROPEAN SEARCH REPORT
## ON EUROPEAN PATENT APPLICATION NO.

EP 20 19 7505

This annex lists the patent family members relating to the patent documents cited in the above-mentioned European search report.
The members are as contained in the European Patent Office EDP file on
The European Patent Office is in no way liable for these particulars which are merely given for the purpose of information.

21-12-2020

| Patent document cited in search report | | Publication date | Patent family member(s) | | Publication date |
|---|---|---|---|---|---|
| CN 103011240 | A | 03-04-2013 | NONE | | |
| US 2002017635 | A1 | 14-02-2002 | NONE | | |
| US 2007149405 | A1 | 28-06-2007 | AU 2003298800 | A1 | 23-06-2004 |
| | | | EP 1567451 | A2 | 31-08-2005 |
| | | | US 2007149405 | A1 | 28-06-2007 |
| | | | WO 2004050558 | A2 | 17-06-2004 |
| | | | ZA 200504782 | B | 26-04-2006 |

EPO FORM P0459

For more details about this annex : see Official Journal of the European Patent Office, No. 12/82

REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP 11503119 PCT
- JP 61004529 A
- JP 2002102335 A

**Non-patent literature cited in the description**

- **TAKASHI SHIGEMATSU et al.** Bone biopsy test. Bayer Yakuhin, Ltd, 2009
- **KAZUHIKO ISHIKIRIYAMA et al.** *JOURNAL OF COLLOID AND INTERFACE SCIENCE,* 1995, vol. 171, 103-111